# EUROPEAN PATENT APPLICATION

(11) **EP 4 071 236 A1**
(43) Date of publication of application: **12.10.2022**
(21) Application number: 21382290.1
(22) Date of filing: 06.04.2021
(51) Int. Cl.: C12N 1/20, A61K 35/747

(54) **PROBIOTIC FOR TREATING AND PREVENTING ALOPECIA**

(71) Applicant: CNCE INNOVACION, S.L., 08015 Barcelona (ES)
(72) Inventor: VILANOVA SERRADOR, Cristina, 08015 Barcelona (ES); PORCAR MIRALLES, Manuel, 08015 Barcelona (ES); MORÁN REY, Francisco Javier, 08015 Barcelona (ES)
(74) Representative: Isern Patentes y Marcas S.L.

(57) **Abstract**

The present invention falls within the general field of probiotics, and in particular, it relates to the new strains of *Lactiplantibacillus* and the use thereof in a suitable composition for treating alopecia as well as the use thereof in a probiotic which comprises at least one strain of *Lactiplantibacillus* selected from *Lactiplantibacillus plantarum* CECT 30102, *Lactiplantibacillus plantarum* CECT 30103 and *Lactiplantibacillus pentosus* CECT 30104.

## Description

### Field of the invention

The present invention falls within the general field of probiotics, and in particular, it relates to a probiotic comprising at least one strain of *Lactiplantibacillus* selected from *Lactiplantibacillus plantarum* CECT 30102, *Lactiplantibacillus plantarum* CECT 30103 and *Lactiplantibacillus pentosus* CECT 30104.

### State of the Art

Hair loss is one of the biggest cosmetic concerns in our society, in Spain alone the sale of nutricosmetic products for treating alopecia occupies a market of more than 57 million euros.

In addition to genetic predisposition, there are other factors related to nutrition that determine the development of alopecia. For this reason, to date, most products for alopecia are based on supplemental vitamins and other essential biomolecules, as well as natural plant extracts that are capable of enhancing hair growth or preventing it from falling out under certain conditions.

In addition to the relevance of dihydrotestosterone in the development of androgenic alopecia, there is another key molecule related to hair health: biotin. Biotin (also known as vitamin B7 or vitamin H) is a water-soluble vitamin that serves as an essential cofactor for carboxylase enzymes in multiple metabolic pathways. The role of biotin in protein synthesis and, more specifically, in keratin production, explains the contribution thereof to healthy nail and hair growth. Current recommendations for biotin in the European Union (Regulation 1169/2011) state that the appropriate daily intake for adults is 50 µg/day. Most healthy people meet these requirements through a well-balanced diet.

Acquired biotin deficiency can occur in several cases, in addition to cases wherein dietary intake is insufficient. A commonly documented cause of acquired biotin deficiency is related to increased consumption of raw eggs. The protein avidin, which is found in raw egg whites, can be denatured by means of cooking, but when it is not cooked, this protein binds to biotin preventing it from being used as an essential cofactor. Patients taking anticonvulsant medications, such as valproic acid, can also show biotin deficiency, which leads these patients to be prophylactically treated with biotin. Additional causes of acquired biotin deficiency include states of alcoholism or pregnancy, use of other medications, such as isotretinoin, alteration of intestinal absorption or prolonged use of antibiotics that modify the normal intestinal flora. Typical symptoms of biotin deficiency include alopecia, eczematous skin rashes, seborrheic dermatitis, conjunctivitis and multiple neurological symptoms, such as depression, lethargy, hypotonia and convulsions.

Due to the relatively low cost thereof and the abundance of cosmetic products that comprise this active ingredient, biotin has become the new trend for consumers who wish to have longer, stronger and healthier hair and nails.

Recently, a new factor involved in the development of alopecia has been discovered: the intestinal microbiome (Immunology and Microbiology. "Probiotics and Prebiotics in Human Nutrition and Health", book edited by Venketeshwer Rao and Leticia G. Rao). In this sense, in recent years, some studies have been carried out with new probiotics belonging to the genus Lactobacillus for promoting hair growth, however, the results were not entirely satisfactory.

On the other hand, the alopecia boosting effect of bacterial species present in the digestive tract of model animals has also been described. Said species are capable of eliminating biotin, and therefore reducing the absorption thereof in the intestine.

Thus, according to Hayashi, Atsushi et al. "Intestinal Dysbiosis and Biotin Deprivation Induce Alopecia through Overgrowth of Lactobacillus murinus in Mice". Cell Reports, Volume 20, Issue 7, 1513 - 1524, alterations in the microbiota can trigger the development of alopecia, especially if the proliferation of intestinal bacteria capable of degrading biotin occurs in the intestinal tract.

The species *Lactobacillus plantarum,* recently taxonomically called *Lactiplantibacillus plantarum* (Jinshui Zheng, et al. A taxonomic note on the genus Lactobacillus: Description of novel genera, emended description of the genus Lactobacillus Beijerinck 1901, and union of Lactobacillaceae and Leuconostocaceae. Int. J. Syst. Evol. Microbiol. 2020) has been used as a probiotic given the beneficial effects it has on the human intestinal microbiota. However, most of the strains belonging to said species have a high capacity to degrade biotin in the human intestine.

There is, therefore, the need to provide a product for treating and preventing alopecia, which has a beneficial effect on the human intestinal microbiota and is effective in the treatment and the prevention of alopecia, which promotes hair generation, hair growth and hair density, as well as ensuring the safety and good tolerance thereof on the part of the subjects to be treated.

### Brief description of the invention

The present invention solves the problems described in the state of the art as it provides a probiotic which, on one hand, does not have the capacity to degrade biotin, and, on the other hand, shifts but does not inhibit the proliferation of biotin-degrading microbial species that are present in the digestive tract, thus improving the availability of biotin in the intestinal tract of alopecic individuals and enabling at the same time a positive effect of the biotin-degrading species on the intestinal microbiota. Said probiotic is effective in the treatment and the prevention of alopecia and promotes hair generation, hair growth and hair density. Likewise, the probiotic of the invention has good safety and good tolerance on the part of the subjects to be treated.

Thus, in a first aspect, the present invention relates to a strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104.

The strain *Lactiplantibacillus plantarum* CECT 30102, (hereinafter strain of the present invention CECT 30102) was deposited by the applicant on 26 May 2020 under the Budapest Treaty in the Spanish Type Culture Collection (CECT). The assigned number was CECT30102.

*Lactiplantibacillus plantarum* CECT 30103 (hereinafter strain of the present invention CECT 30103) was deposited by the applicant on 26 May 2020 under the Budapest Treaty in the Spanish Type Culture Collection (CECT). The assigned number was CECT30103.

*Lactiplantibacillus plantarum* CECT 30104 (hereinafter strain of the present invention CECT 30104) was deposited by the applicant on 26 May 2020 under the Budapest Treaty in the Spanish Type Culture Collection (CECT). The assigned number was CECT30104.

The strains of the first aspect have the same growth capacity as the degrading strains.

The second aspect of the present invention relates to a composition comprising at least one strain *Lactiplantibacillus* selected from the list consisting of the strain of the present invention CECT 30102, the strain of the present invention CECT 3010, and the strain of the present invention CECT 30104; and at least one excipient.

The third aspect of the present invention relates to a strain of *Lactiplantibacillus* according to the first aspect, selected from among *Lactiplantibacillus plantarum* CECT 30102, *Lactiplantibacillus plantarum* CECT 30103 and/or *Lactiplantibacillus pentosus* CECT 30104, for use in the treatment and/or the prevention of alopecia.

The fourth aspect of the invention is related to the composition of the second aspect of the invention for use in the treatment and/or the prevention of alopecia.

The fifth aspect of the invention is related to the composition of the second aspect of the invention for use as a hair generation promoting agent, of hair growth promoting agent and/or as a hair density promoting agent.

The sixth aspect of the invention is related to a probiotic comprising at least one strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104, or comprising the composition of the second aspect, for use in the treatment and/or the prevention of alopecia.

The seventh aspect of the invention is related to a probiotic comprising at least one strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104, or comprising the composition of the second aspect, for use as a hair generation promoting agent, of hair growth promoting agent and/or as a hair density promoting agent.

The eighth aspect of the invention is related to a probiotic comprising at least one strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104, or comprising the composition of the second aspect, for use in the treatment and/or the prevention of alopecia comprising the administration of at least one unit of probiotic of the seventh aspect or at least one unit per day of the composition of the second aspect, preferably at least one unit of probiotic comprises between 50 and 200 mg of the strain of the first aspect or one unit of the composition of the second aspect comprising between 50 and 200 mg of the strain of the first aspect.

### Detailed description of the invention

In a first aspect, the present invention relates to a strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104. The strains of the first aspect of the invention have good capacity for resistance to both gastric and intestinal conditions. Likewise, the strains of the first aspect have the same growth capacity as the biotin degrading strains, so that the intake thereof as probiotics in the usual amounts for treating alopecia (at least 10⁶ CFUs/day) cause a shift in the degrading populations in the human intestine.

The strain *Lactiplantibacillus plantarum* CECT 30102, (hereinafter strain of the present invention CECT 30102) was deposited by the applicant on 26 May 2020 under the Budapest Treaty in the Spanish Type Culture Collection (CECT). The assigned number was CECT30102.

*Lactiplantibacillus plantarum* CECT 30103 (hereinafter strain of the present invention CECT 30103) was deposited by the applicant on 26 May 2020 under the Budapest Treaty in the Spanish Type Culture Collection (CECT). The assigned number was CECT30103.

*Lactiplantibacillus plantarum* CECT 30104 (hereinafter strain of the present invention CECT 30104) was deposited by the applicant on 26 May 2020 under the Budapest Treaty in the Spanish Type Culture Collection (CECT). The assigned number was CECT30104.

The second aspect of the present invention relates to a composition comprising at least one strain *Lactiplantibacillus* selected from the list consisting of the strain of the present invention CECT 30102, the strain of the present invention CECT 30103 and the strain of the present invention CECT 30104; and at least one excipient.

In the present invention, "composition" refers to a set of components formed by at least one of the strains of the present invention, in any concentration.

The composition was effective both with the presence of one strain and with the presence of the combination of 2 strains, and with the combination of 3 strains. Preferably, the composition of the second aspect comprises at least the strain of the present invention CECT 30103 and/or the strain of the present invention CECT 30104.

In the context of the present invention, the term "excipient" refers to any substance which, included in the composition, helps to control the physico-chemical and organoleptic properties, dosage and bioavailability thereof, such as absorption, stabilisation, consistency, taste and/or smell.

In another particular embodiment of the second aspect of the invention, the excipient can be selected from among: preservatives, thickeners, stabilisers, flavourings, dyes, diluents, lubricants, binders, protectors, humectants and/or disintegrating agents, without excluding other types of excipients not mentioned herein.

In a preferred embodiment, the composition of the second aspect comprises at least one strain *Lactiplantibacillus* selected from the list consisting of the strain of the present invention CECT 30102, the strain of the present invention CECT 30103 and the strain of the present invention CECT 30104; and at least one diluent and one lubricant.

In a preferred embodiment, the composition of the second aspect comprises at least one strain Lactiplantibacillus selected from the list consisting of the strain of the present invention CECT 30102, the strain of the present invention CECT 30103, and the strain of the present invention CECT 30104; wherein the total concentration by weight of the strain Lactiplantibacillus is in a range from 99.9 to 20% and at least one excipient in a concentration by weight in a range from 0.1% to 80%. The percentage by weight of the concentration of the strain Lactiplantibacillus includes the total concentration of the strains present; in the event that there is only one strain, the total concentration will correspond to the concentration of that strain. If there were two strains according to the strain of the first aspect, the total concentration would be the sum of the two. Likewise, if there were 3 strains, it would be the sum of the three.

In a preferred embodiment, the composition of the second aspect comprises at least one strain Lactiplantibacillus selected from the list consisting of the strain of the present invention CECT 30102, the strain of the present invention CECT 30103, and the strain of the present invention CECT 30104; wherein the total concentration by weight of the strain Lactiplantibacillus is in a range from 80 to 20%; and at least one excipient in a concentration by weight in a range from 20% to 80%.

In a more preferred embodiment, the composition of the second aspect comprises at least one strain Lactiplantibacillus selected from the list consisting of the strain of the present invention CECT 30102, the strain of the present invention CECT 30103 and the strain of the present invention CECT 30104, in a concentration by weight in a range from 60 to 30%; and at least one excipient in a concentration by weight in a range from 40% to 70%.

In a more preferred embodiment, the composition of the second aspect comprises at least one strain Lactiplantibacillus selected from the list consisting of the strain of the present invention CECT 30102, the strain of the present invention CECT 30103 and the strain of the present invention CECT 30104, in a concentration by weight in a range from 60 to 30%, and excipients in a concentration by weight in a range from 40% to 70%, wherein the excipients comprise at least a diluent and a lubricant and optionally a thickener. Preferably wherein the diluent is in a concentration by weight with respect to the total composition of the first aspect from 10-30%, the lubricant from 10% to 30% and optionally the thickener in a range from 8-13%.

In a preferred embodiment, the lubricants are selected from the list consisting of: magnesium stearate, calcium stearate, zinc stearate, talc, fatty acids and esters thereof; preferably the fatty acids are selected from myristic acid, palmitic acid and/or stearic acid.

In a preferred embodiment, the diluents are selected from the list consisting of: microcrystalline cellulose, corn starch, hydrolysed starch and partially pregelatinised starches, anhydrous lactose, lactose monohydrate and selected alcohols forming sorbitol, xylitol and/or mannito

In a preferred embodiment, the disintegrating agents are selected from the list consisting of: sodium starch glycolate, crospovidone, croscarmellose, sodium croscarmellose, gellan gum, xanthan gum and soy polysaccharide.

In a preferred embodiment, the stabilisers are selected from the list consisting of: sodium benzoate, citric acid, Vitamin E, Vitamin C, Butylated hydroxyanisole (BHA), Butylated hydroxytoluene (BHT), sodium ethylenediamine tetra-acetic acid (EDTA),

In a preferred embodiment of the second aspect of the invention, the composition of the present invention is a composition that is administered orally or topically, preferably it is a composition that is administered orally.

In a particular embodiment, the composition of the second aspect is selected from the list consisting of solid, semi-solid, liquid and gel form.

In a preferred embodiment, the composition of the second aspect is selected from the list consisting of capsules, tablets, powder and granules.

In a more preferred embodiment, the composition of the second aspect comprises at least one strain according to the strains of the first aspect of the invention and excipients, wherein the excipients comprise silicates, preferably sodium silicates, calcium, potassium, magnesium or hydrophilic fumed silica; magnesium stearate and microcrystalline cellulose.

In a particular embodiment, the composition of the present invention is a pharmaceutical composition. In the present invention, the term "pharmaceutical composition" refers to a set of components comprising at least one of the strains of the present invention, in any concentration and having at least one application for improving the physical or physiological condition of the subject. In a particular embodiment, the pharmaceutical composition can be a medication.

In a particular embodiment, the composition of the present invention is a cosmetic composition. In the present invention, the term "cosmetic composition" refers to a set of components comprising at least one of the strains of the present invention, in any concentration and having a function of cleaning, beautifying and/or altering the physical appearance of the subject.

In a preferred embodiment, the composition of the present invention is a nutritional composition. In the present invention, the term "nutritional composition" refers to a set of components comprising at least one of the strains of the present invention, in any concentration and included in at least one food or food ingredient, such that, regardless of providing nutrients to the subject who ingests it, it provides a better physical or physiological state of the subject.

In a preferred embodiment, the nutritional composition is a food, a dietary supplement, a food supplement, a probiotic or a nutraceutical.

In a preferred embodiment, the composition of the present invention has a concentration of the strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104, of at least 10⁴ colony forming units per gram (CFU/g) of final composition, more preferably the concentration is in a range from 10⁶ to 10¹¹. The amount of 10⁴ colony forming units is equivalent to at least 10⁴ live bacteria.

The third aspect of the present invention relates to a strain of *Lactiplantibacillus* according to the first aspect, selected from among *Lactiplantibacillus plantarum* CECT 30102, *Lactiplantibacillus plantarum* CECT 30103 and/or *Lactiplantibacillus pentosus* CECT 30104, for use in treatment and/or the prevention of alopecia or for use as a hair generation promoting agent, of hair growth promoting agent and/or as a hair density promoting agent.

The fourth aspect of the invention is related to the composition of the second aspect of the invention for use in treatment and/or prevention of alopecia.

The fifth aspect of the invention is related to the composition of the second aspect of the invention for use as a hair generation promoting agent, of hair growth promoting agent and/or as a hair density promoting agent.

In the present invention, the term "treatment" refers to combating the effects of the pathological condition of interest in the subject.

In the present invention, the term "prevention" refers to preventing the onset of the pathological condition of interest in the subject.

The sixth aspect of the invention is related to a probiotic comprising at least one strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104, or comprising the composition of the second aspect, for use in the treatment and/or the preventionof alopecia.

The seventh aspect of the invention is related to a probiotic comprising at least one strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104, or comprising the composition of the second aspect, use as a hair generation promoting agent, of hair growth promoting agent and/or as a hair density promoting agent.

In a preferred embodiment, the probiotic of the seventh aspect comprises between 50 and 200 mg of the strain of the first aspect or a unit of the composition of the second aspect comprising between 50 and 200 mg of the strain of the first aspect.

In another preferred embodiment, the probiotic of the seventh aspect comprises a composition wherein the strain of *Lactiplantibacillus* is selected from among *Lactiplantibacillus plantarum* CECT 30102, *Lactiplantibacillus plantarum* CECT 30103 and/or *Lactiplantibacillus pentosus* CECT 30104, and the concentration thereof to the final composition is at least 10⁴ colony-forming units (CFU/g), more preferably the concentration is in a range from 10¹¹ to 10⁶ The amount of 10⁴ colony-forming units is equivalent to at least 10⁴ live bacteria.

In the present invention, "probiotic" refers to live microorganisms, which, when administered in adequate amounts, confer a health benefit to the host, said benefit can be physical or physiological.

The eighth aspect of the invention is related to a probiotic comprising at least one strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104, or comprising the composition of the second aspect, for use in treatment and/or the prevention of alopecia comprising the administration of at least one unit of probiotic per day or at least one unit per day of the composition of the second aspect, preferably at least one unit of probiotic comprises between 50 and 200 mg of the strain of the first aspect or one unit of the composition of the second aspect comprising between 50 and 200 mg of the strain of the first aspect.

### Examples

### Example 1: Isolation and identification of the strains

The biological material object of the present invention was isolated from the processing of stool samples from volunteer individuals with different degrees of alopecia (Types III, V and VI, according to the Hamilton classification). The samples were taken in sterile containers and processed immediately.

To isolate microorganisms from the stool samples, culture media compatible with the subsequent quantification of biotin degradation (MRS medium (Table 1) and minimal saline medium with biotin as the sole carbon source (Table 2)) were used.

**Table 1: MRS medium composition**

| INGREDIENT | CONCENTRATION (g/l) |
|---|---|
| Peptone | 10 |
| Meat extract | 10 |
| Yeast extract | 4 |
| Glucose | 20 |
| Sodium acetate | 5 |
| Tween 80 | 1 |
| K₂HPO₄·3H₂0 | 2 |
| MgSO₄ | 0.2 |
| MnSO₄ | 0.05 |
| Agar | 15 |

**Table 2: Composition of the minimal medium with biotin as a carbon source**

| INGREDIENT | CONCENTRATION (g/l) |
|---|---|
| NH₄H₂PO₄ | 1 |
| KCI | 0.2 |
| MgSO₄ • 7H₂O | 1 |
| Biotin | variable |
| Agar | 15 |

Once seeded, the plates were incubated for 72h at 37°C under microaerophilic conditions (<5% oxygen). Among the colonies that grew, the strains *Lactiplantibacillus plantarum* CECT 30102, *Lactiplantibacillus plantarum* CECT 30103 and/or *Lactiplantibacillus pentosus* CECT 30104 were isolated.

The identification of said isolates was carried out by partial sequencing of the 16S rRNA gene, using universal primers. In summary, the individual colonies were resuspended in 100 µl of sterile water and boiled for 5 min. Subsequently, 1 µl of the suspension was added to the PCR mix (primers, 0.1-1 µM; MgCI2, 1.5-3 mM; dNTPs, 0.2 mM; and 0.25-2.5 U DNA polymerase). The amplification conditions were as follows for all cases: 5 min denaturation at 94°C, followed by 25 cycles of amplification consisting of 1 min denaturation at 94°C, 45 s annealing at 49°C, and 2 min extension at 72°C. Lastly, an extra extension step of 10 min at 72°C was included.

### Example 2: Genetic characterisation of the strain Lactiplantibacillus plantarum CECT 30102

To verify the genomic exclusivity of the strain *Lactiplantibacillus plantarum* CECT 30102, a study was carried out *in silico* using different biocomputing tools. First, genomic similarity, measured as MASH distances, between the strain *Lactiplantibacillus plantarum* CECT 30102 and all strains belonging to the species *Lactobacillus plantarum,* the genomes of which were available in public databases (NCBI and PATRIC; Wattan *et al.,* 2014) dated 15 June 2020, were analysed. This analysis was performed using the Mash/MinHash tool (Ondov *et al.,* 2016).

Next, the Average Nucleotide Index (ANI) between the strain *Lactiplantibacillus plantarum* CECT 30102 and the 10 most similar strains of Lactobacillus plantarum at the genomic level was calculated. This comparison was made with the JSpeciesWS tool (Richter *et al.,* 2016).

Finally, a phylogenetic tree of the strain *Lactiplantibacillus plantarum* CECT 30102 was obtained based on genomic data (=phylogenomic). The phylogenomic tree was analysed including the 50 strains of *L. plantarum* most similar to *Lactiplantibacillus plantarum* CECT 30102. The phylogenomic tree was based on the concatenation of 500 single copy orthologous genes in the genome. The concatenated amino acid sequences were subsequently aligned with the MUSCLE tool (Edgar *et al.,* 2004), inferring a phylogenetic tree by using the method of maximum likelihood (ML) with the RAxML tool (Stamatakis *et al.,* 2014). The robustness of the phylogenetic inference was estimated using bootstrap values after 100 replicates.

According to the genomic similarity values dDDH and ANI, the strain *Lactiplantibacillus plantarum* CECT 30102 belongs to the species *Lactobacillus plantarum.* This bacterial species is recognised by the European Food Safety Authority (EFSA) as a Qualified Presumption of Safety (QPS) species (Koutsoumanis *et al.,* 2019) and by the United States Food and Drug Administration (FDA) as GRAS (Generally Regarded As Safe). Accordingly, it can be legally used as a food supplement.

By way of an informative note, the species *Lactobacillus plantarum* has been recently reclassified as another genus with the name *Lactiplantibacillus plantarum* (Zheng *et al.,* 2020). Both names will be used interchangeably in the present invention. Example 3: *Genetic characterisation of the strain Lactiplantibacillus plantarum CECT 30103*

The procedure was carried out according to the methodology explained in example 2, but for the strain *Lactiplantibacillus plantarum CECT 30103.*

Once the strains of *Lactobacillus plantarum* most similar to the strain *Lactiplantibacillus plantarum CECT 30103* were identified at the genomic level, a phylogenomic tree was obtained to determine the phylogenetic relationships thereof and the evolution rate of the different lineages. The phylogenomic tree was inferred with the 126 strains of *L. plantarum* most similar to *Lactiplantibacillus plantarum CECT 30103.* The phylogenomic tree was obtained after concatenating 476 single copy orthologous genes in the genome. The concatenated amino acid sequences were aligned with the MUSCLE tool (Edgar *et al.,* 2004), subsequently inferring a phylogenetic tree by using the method of maximum likelihood (ML) with the RAxML tool (Stamatakis *et al.,* 2014). The robustness of the phylogenetic inference was estimated using bootstrap values after 100 replicates.

Next, the JSpeciesWS Average Nucleotide Index (ANI) tool (Richter *et al.,* 2016) was used to determine the percentage of genomic similarity between genomes in order to be able to thereby corroborate the exclusivity of the identity of the strain *Lactiplantibacillus plantarum CECT 30103.*

The first part of the study consisted of ordering the 730 strains of *L. plantarum* available based on their genomic similarity with respect to *Lactiplantibacillus plantarum CECT 30103.* For this, Mash distances were used. The lowest Mash distance (and therefore the most similar to *Lactiplantibacillus plantarum CECT 30103)* was obtained with the strainsSRCM103297, *IPLA88 and KMB_621.* The strain *L. plantarum SRCM103297* was isolated from food in South Korea in 2018; the sourdough strain IPLA88 and the Bryndza cheese strain KMB_621 in Slovakia in 2008.

Given the phylogenomic tree based on 476 concatenated genes, the strain *Lactiplantibacillus plantarum CECT 30103* formed a monophyletic group with the strains SRCM103297, IPLA88 and KMB_621. These results coincided with those obtained based on the Mash distances, corroborating the identity of the bacterial strains most similar to *Lactiplantibacillus plantarum CECT 30103.* This grouping showed a bootstrap value of 100% indicating that the phylogenetic inference obtained was stable. Furthermore, the strain *Lactiplantibacillus plantarum CECT 30103* appears forming an independent branch within the cluster, indicating that the genome thereof differs from the phylogenetically closest neighbours thereof.

Lastly, to numerically quantify the genomic differences between the strain *Lactiplantibacillus plantarum CECT 30103* and the phylogenetically closest neighbours thereof, the ANI (Average Nucleotide Index) value was calculated. The results showed that the genomic similarity between the strain *Lactiplantibacillus plantarum CECT 30103* and the closest neighbours thereof was less than 99.35% and therefore they have a variability in the genomic content thereof.

### Example 4: Genetic characterisation of the strain Lactiplantibacillus pentosus CECT 30104

The procedure was carried out according to the methodology explained in example 2, but for the strain *Lactiplantibacillus pentosus* CECT 30104.

According to the genomic similarity values dDDH and ANI, the strain *Lactiplantibacillus pentosus* CECT 30104 belongs to the species *Lactobacillus plantarum.* This bacterial species is recognised by the European Food Safety Authority (EFSA) as a Qualified Presumption of Safety (QPS) species (Koutsoumanis *et al.,* 2019) and by the United States Food and Drug Administration (FDA) as GRAS (Generally Regarded As Safe). Accordingly, it can be legally used as a food supplement.

There are a total of 531 genomes of different strains of *L. plantarum* in the public databases. To determine which strains existing up to now are the most similar at the genomic level with respect to strain *Lactiplantibacillus pentosus* CECT 30104, Mash distances were calculated. The most similar strains at the genomic level were *L*. *plantarum LM1004 kimchi* (CP025988) isolated from cabbage kimchi, a garnish used in Korea, followed by the strain *L. plantarum NRCC1* (LUSM00000000) isolated from the stomach of a camel in India.

To more precisely determine the degree of similarity between the strain *Lactiplantibacillus pentosus* CECT 30104 and the 10 most similar strains of *L. plantarum* based on the Mash distances, the genomic similarity index was calculated (Average Nucleotide Identity; Judge *et al.,* 2016). The greatest similarity of the genomic sequence was obtained with the strain *L. plantarum* NRCC1 (ANI = 99.15%). This result confirms that a portion of the genome of the strain *Lactiplantibacillus pentosus* CECT 30104 is exclusive.

Lastly, the phylogenomic tree confirmed that the strain belongs to the species L. *plantarum,* the strain pc-26 (isolated from human stool) phylogenetically being the closest to strain *Lactiplantibacillus pentosus* CECT 30104, although with a certain distance.

### Example 5: Characterisation of inability to degrade biotin

Determining the capacity to degrade biotin was done by using the rich medium method (MRS) with increasing concentrations of biotin. In this method, for each of the strains isolated in conventional MRS medium (Table 1), the growth obtained in MRS medium without biotin and with increasing concentrations of biotin was compared, as seen in Table 3. The strains with degradation capacity were characterised by linearly increasing the growth thereof as the concentration of biotin increased, while the non-degrading strains did not present significant differences in the growth thereof in the different media. Likewise, a greater slope in the growth line indicates a greater degradation capacity, and vice versa. The fine-tuning of this medium was carried out with the standard strain *L. plantarum* CECT 220, with known capacity to degrade biotin (Table 3).

As observed in Table 3, while the reference strain strongly increases the growth thereof as the concentration of biotin increases in the medium, the strains of the present invention have a very low growth increase (gentler slope in Table 3a) or practically null growth, which suggests that there is no significant biotin degradation on their part. Table 3a displays the slope values (directly related to the degradation capacity) and the linearity adjustment (R²) obtained for each strain:

**Table 3: Biotin degradation values measured as concentration versus growth (OD600) of the selected strains and of the degrading strain used as control**

| Strain | Biotin concentration | Growth (OD600) |
|---|---|---|
| *Lactiplantibacillus pentosus* CECT 30104 | 0 | 0.08 |
| | 0.1 | |
| | 0.2 | 0.4 |
| | 0.3 | 0.4 |
| *Lactiplantibacillus plantarum* CECT 30103 | | |
| | 0 | 0.13 |
| | 0.1 | 0.42 |
| | 0.2 | 0.49 |
| | 0.3 | 0.47 |
| *Lactiplantibacillus plantarum* CECT 30102 | | |
| | 0 | 0.16 |
| | 0.1 | 0.42 |
| | 0.2 | 0.28 |
| | 0.3 | 0.38 |
| Positive control (degrading strain) | | |
| | 0 | 0.23 |
| | 0.1 | 0.7 |
| | 0.2 | 0.88 |
| | 0.3 | 0.98 |

**Table 3a: Biotin degradation values measured as slope of the adjustment line of the selected strains and of the degrading strain used as control.**

| Strain | Slope | Adjustment (R² value) |
|---|---|---|
| *Lactiplantibacillus pentosus* CECT 30104 | 0.952 | 0.652 |
| *Lactiplantibacillus plantarum* CECT 30103 | 0.214 | 0.652 |
| *Lactiplantibacillus plantarum* CECT 30102 | 0.019 | 0.049 |
| Positive control (degrading strain) | 2.297 | 0.869 |

### Example 6: Growth capacity with respect to degrading strains

To compare the growth of different strains, assays were performed to measure the intensity of growth with respect to the main biotin-degrading strains. This was done in order to verify if they had a similar or higher growth rate than the degrading ones, which would facilitate a shift in the population . For this, cultures were prepared in MRS medium (50 ml) and inoculated from an initial OD of 0.1. After incubation of the cultures at 37°C for 18 h, OD600 (optical density) was measured on a spectrophotometer. Three independent cultures of each strain were carried out, and the existence of statistically significant differences in growth was verified for each pair of strains.

Table 4 shows a comparison of the growth of the three selected strains with respect to a selection of biotin-degrading strains isolated from human intestine samples. In light of these results, the strains of the present invention have the same growth capacity as the degrading strains, so the intake thereof as probiotics in the usual amounts in treatments of this type (amounts of 10⁶ CFUs/day) produces a shifting of the degrading populations in the human intestine.

**Table 4: Comparison of the growth of biotin degrading strains and the strains of the present invention.**

| Strain | OD600 |
|---|---|
| *Lactiplantibacillus pentosus* CECT 30104 | 12.2 |
| *Lactiplantibacillus plantarum* CECT 30103 | 14.2 |
| *Lactiplantibacillus plantarum* CECT 30102 | 13.1 |
| *Lactiplantibacillus plantarum DCn1_2 (degrading strain)* | 10.2 |
| *Lactiplantibacillus plantarum DCn1_15 (degrading strain)* | 11.8 |
| *Lactiplantibacillus plantarum DCn1_9 (degrading strain)* | 12.6 |

### Example 7: Resistance to gastrointestinal conditions

In order to verify that the selected strains can survive passage through the gastrointestinal tract, and therefore carry out their activity therein, the resistance assays described above in the methodology were carried out.

Table 5 summarises the results obtained for the three selected strains:

**Table 5: Resistance of the selected strains to passage through the gastric and intestinal tracts.**

| Strain | Gastric tract resistance | Intestinal tract resistance |
|---|---|---|
| *Lactiplantibacillus pentosus* CECT 30104 | ++ | +++ |
| *Lactiplantibacillus plantarum* CECT 30103 | +++ | +++ |
| *Lactiplantibacillus plantarum* CECT 30102 | +++ | +++ |

| | | |
|---|---|---|
| +: survival greater than 50% and less than 70% ++: survival greater than 70% and less than 90% +++: survival greater than 90% | | |

The data shows that the strains of the present invention had an outstanding ability to resist both gastric and intestinal conditions.

Based on the results shown herein, it is shown that the strains of the first aspect of the invention are original and do not duplicate any of the strains deposited in databases, which are not biotin-degrading strains and which furthermore shift, but do not inhibit biotin-degrading strains that exist in the intestinal tract.

### Example 8: Compositions of the present invention

To prepare the composition of the present invention, each of the components were weighed separately. Subsequently, each one of the components were mixed according to the composition of Table 6, until obtaining a powder mixture, which was then encapsulated using procedures commonly used in laboratories for the processing and encapsulation.

**Table 6: Example of a composition of the present invention in capsule form**

| INGREDIENTS % BY WEIGHT IN THE COMPOSITION | |
|---|---|
| Strain of the present invention | 42-46% by weight |
| Hydrophilic fumed silica (Aerosil) | 10-12% by weight |
| Magnesium stearate (anti-caking agent). | 21-23% by weight |
| Microcrystalline cellulose (filler) | 21-23% by weight |

Next, different compositions were prepared, table 7, using the same method described above, changing the amounts of each component.

**Table 7 shows 4 examples of the composition of the present invention.**

| | COMPOSITION 1 | COMPOSITION 2 | COMPOSITION 3 | COMPOSITION 4 |
|---|---|---|---|---|
| | % by weight | % by weight | % by weight | % by weight |
| *Lactiplantibacillus plantarum* CECT 30102 | 42 | - | - | 15 |
| *Lactiplantibacillus plantarum* CECT 30103 | - | 44 | - | 15 |
| *Lactiplantibacillus pentosus* CECT 30104 | - | - | 46 | 16 |
| Aerosil | 11.6 | 11 | 11 | 10 |
| Magnesium stearate | 23 | 23 | 22 | 21 |
| Microcrystalline cellulose | 23 | 22 | 21 | 23 |

### Example 9: Compositions of the present invention

The effect of the composition of the capsule of example 8 was evaluated, in the capillary density of subjects with androgenic alopecia, after administering said capsule once a day for 6 weeks, compared to a group of subjects given a placebo.

The results of the study showed an increase in relevant hair per centimetre and a better hair thickness compared to the placebo.

The study also showed that a high number of follicular units and a low level of sebum were formed in the composition of the invention, as well as good safety and tolerance.

>DCn1_05
ATACATGCAAGTCGAACGAACTCTGGTATTGATTGGTGCTTGCATCATGATTTACATTTGAGTGAGTGGCGAACTGG
TGAGTAACACGTGGGAAACCTGCCCAGAAGCGGGGGATAACACCTGGAAACAGATGCTAATACCGCATAACAACTTG
GACCGCATGGTCCGAGTTTGAAAGATGGCTTCGGCTATCACTTTTGGATGGTCCCGCGGCGTATTAGCTAGATGGTG
GGGTAACGGCTCACCATGGCAATGATACGTAGCCGACCTGAGAGGGTAATCGGCCACATTGGGACTGAGACACGGCC
CAAACTCCTACGGGAGGCAGCAGTAGGGAATCTTCCACAATGGACGAAAGTCTGATGGAGCAACGCCGCGTGAGTGA
AGAAGGGTTTCGGCTCGTAAAACTCTGTTGTTAAAGAAGAACATATCTGAGAGTAACTGTTCAGGTATTGACGGTAT
TTAACCAGAAAGCCACGGCTAACTACGTGCCAGCAGCCGCGGTAATACGTAGGTGGCAAGCGTTGTCCGGATTTATT
GGGCGTAAAGCGAGCGCAGGCGGTTTTTTAAGTCTGATGTGAAAGCCTTCGGCTCAACCGAAGAAGTGCATCGGAAA
CTGGGAAACTTGAGTGCAGAAGAGGACAGTGGAACTCCATGTGTAGCGGTGAAATGCGTAGATATATGGAAGAACAC
CAGTGGCGAAGGCGGCTGTCTGGTCTGTAACTGACGCTGAGGCTCGAAAGTATGGGTAGCAAACAGGATTAGATACC
CTGGTAGTCCATACCGTAAACGATGAATGCTAAGTGTTGGAGGGTTTCCGCCCTTCAGTGCTGCAGCTAACGCATTA
AGCATTCCGCCTGGGGAGTACGGCCGCAAGGCTGAAACTCAAAGGAATTGACGGGGGCCCGCACAAGCGGTGGAGCA
TGTGGTTTAATTCGAAGCTACGCGAAGAACCTTACCAGGTCTTGACATACTATGCAAATCTAAGAGATTAGACGTTC
CCTTCGGGGACATGGATACAGGTGGTGCATGGTTGTCGTCAGCTCGTGTCGTGAGATGTTGGGTTAAGTCCCGCAAC
GAGCGCAACCCTTATTATCAGTTGCCAGCATTAAGTTGGGCACTCTGGTGAGACTGCCGGTGACAAACCGGAGGAAG
GTGGGGATGACGTCAAATCATCATGCCCCTTATGACCTGGGCTACACACGTGCTACAATGGATGGTACAACGAGTTG
CGAACTCGCGAGAGTAAGCTAATCTCTTAAAGCCATTCTCAGTTCGGATTGTAGGCTGCAACTCGCCTACATGAAGT
CGGAATCGCTAGTAATCGCGGATCAGCATGCCGCGGTGAATACGTTCCCGGGCCTTGTACACACCGCCCGTCACACC
ATGAGAGTTTGTAACACCCAAAGTCGGTGGGGTAACCTTTTAGGAACCAGCCGCCTAAG

>DCn1_06
TCATTAATTTGAGAGTTTGATCCTGGCTCAGGACGAACGCTGGCGGCGTGCCTAATACATGCAAGTCGAACGAACTC
TGGTATTGATTGGTGCTTGCATCATGATTTACATTTGAGTGAGTGGCGAACTGGTGAGTAACACGTGGGAAACCTGC
CCAGAAGCGGGGGATAACACCTGGAAACAGATGCTAATACCGCATAACAACTTGGACCGCATGGTCCGAGTTTGAAA
GATGGCTTCGGCTATCACTTTTGGATGGTCCCGCGGCGTATTAGCTAGATGGTGGGGTAACGGCTCACCATGGCAAT
GATACGTAGCCGACCTGAGAGGGTAATCGGCCACATTGGGACTGAGACACGGCCCAAACTCCTACGGGAGGCAGCAG
TAGGGAATCTTCCACAATGGACGAAAGTCTGATGGAGCAACGCCGCGTGAGTGAAGAAGGGTTTCGGCTCGTAAAAC
TCTGTTGTTAAAGAAGAACATATCTGAGAGTAACTGTTCAGGTATTGACGGTATTTAACCAGAAAGCCACGGCTAAC
TACGTGCCAGCAGCCGCGGTAATACGTAGGTGGCAAGCGTTGTCCGGATTTATTGGGCGTAAAGCGAGCGCAGGCGG
TTTTTTAAGTCTGATGTGAAAGCCTTCGGCTCAACCGAAGAAGTGCATCGGAAACTGGGAAACTTGAGTGCAGAAGA
GGACAGTGGAACTCCATGTGTAGCGGTGAAATGCGTAGATATATGGAAGAACACCAGTGGCGAAGGCGGCTGTCTGG
TCTGTAACTGACGCTGAGGCTCGAAAGTATGGGTAGCAAACAGGATTAGATACCCTGGTAGTCCATACCGTAAACGA
TGAATGCTAAGTGTTGGAGGGTTTCCGCCCTTCAGTGCTGCAGCTAACGCATTAAGCATTCCGCCTGGGGAGTACGG
CCGCAAGGCTGAAACTCAAAGGAATTGACGGGGGCCCGCACAAGCGGTGGAGCATGTGGTTTAATTCGAAGCTACGC
GAAGAACCTTACCAGGTCTTGACATACTATGCAAATCTAAGAGATTAGACGTTCCCTTCGGGGACATGGATACAGGT
GGTGCATGGTTGTCGTCAGCTCGTGTCGTGAGATGTTGGGTTAAGTCCCGCAACGAGCGCAACCCTTATTATCAGTT
GCCAGCATTAAGTTGGGCACTCTGGTGAGACTGCCGGTGACAAACCGGAGGAAGGTGGGGATGACGTCAAATCATCA
TGCCCCTTATGACCTGGGCTACACACGTGCTACAATGGATGGTACAACGAGTTGCGAACTCGCGAGAGTAAGCTAAT
CTCTTAAAGCCATTCTCAGTTCGGATTGTAGGCTGCAACTCGCCTACATGAAGTCGGAATCGCTAGTAATCGCGGAT
CAGCATGCCGCGGTGAATACGTTCCCGGGCCTTGTACACACCGCCCGTCACACCATGAGAGTTTGTAACACCCAAAG
TCGGTGGGGTAACCTTTTAGGAACCAGCCGCCTAAGGTGGGACAGATGATTAGGGTGAAGTCGTAACAAGGTAGCCG
TAGGAGAACCTGCGGCTGGATCACCTCCTTTCTA

>DCn1_07
GCATGGTCCGAGTTTGAAAGATGGCTTCGGCTATCACTTTTGGATGGTCCCGCGGCGTATTAGCTAGATGGTGAGGT
AACGGCTCACCATGGCAATGATACGTAGCCGACCTGAGAGGGTAATCGGCCACTTTGGGACTGAGACAGGGTCAAAA
AATAAACAGGAGGCAGCAGTAGGGAATCTTCCACAATGGACGAAAGTCTGATGGAGCAACGCCGCGTGAGTGAAGAA
GGGTTTCGGCTCGTAAAACTCTTTTGTTAAAGAATAACATATCTGAGAGTAACTGTTCAGGTATTGACGGTATTTAA
CCAGAAAGCCACGGCTAACTACGTGCCAGCAGCCGCGGTAATACGTAGGTGGCAAGCGTTGTCCGGATTTATTGGGC
GTAAAGCGAGCGCAGGCGGTTTTTTAAGTCTGATGTGAAAGCTTTCGGCTCAACCGAAGAAGTGCATCGGAAACTGG
GAAACTTGAGTGCAGAAGAGGACAGTGGAACTCCATGTGTAGCGGTGAAATGCGTAGATATATGGAAGAACACCAGT
GGCGAAGGCGGCTGTCTGGTCTGTAACTGACGCTGAGGCTCGAAAGTATGGGTAGCAAACAGGATTAGATACCCTGG
TAGTCCATACCGTAAACGATGAATGCTAAGTGTTGGAGGGTTTCCGCCCTTCAGTGCTGCAGCTAACGCATTAAGCA
TTCCGCCTGGGGAGTACGGCCGCAAGGCTGAAACTCAAAGGAATTGACGGGGGCCCGCACAAGCGGTGGAGCATGTG
GTTTAATTCGAAGCTACGCGAAGAACCTTACCAGGTCTTGACATACTATGCAAATCTAAGAGATTAGACGTTCCCTT
CGGGGACATGGATACAGGTGGTGCATGGTTGTCGTCAGCTCGTGTCGTGAGATGTTGGGTTAAGTCCCGCAACGAGC
GCAACCCTTATTATCAGTTGCCAGCATTAAGTTGGGCACTCTGGTGAGACTGCCGGTGACAAACCGGAGGAAGGTGG
GGATGACGTCAAATCATCATGCCCCTTATGACCTGGGCTACACACGTGCTACAATGGATGGTACAACGAGTTGCGAA
CTCGCGAGAGTAAGCTAATCTCTTAAAGCCATTCTCAGTTCGGATTGTAGGCTGCAACTCGCCTACATGAAGTCGGA

ATCGCTAGTAATCGCGGATCAGCATGCCGCGGTGAATACGTTCCCGGGCCTTGTACACACCGCCCGTCACACCATGA
GAGTTTGTAACACCCAAAGTCGGTGGGGTAACCTTTTAGGAACCAGCCGCCTAAG

## Claims

1. A strain of *Lactiplantibacillus* selected from among *Lactiplantibacillus plantarum* CECT 30102, *Lactiplantibacillus plantarum* CECT 30103 and/or *Lactiplantibacillus pentosus* CECT 30104.

2. A composition comprising at least one strain of *Lactiplantibacillus* according to claim 1.

3. The composition according to claim 2, which comprises at least one excipient.

4. The composition according to claim 2, wherein the excipient comprises at least one diluent and one lubricant.

5. The composition according to any of claims 2 to 4, wherein the total concentration by weight of the strain Lactiplantibacillus is in a range from 99.9 to 20%, and at least one excipient in a concentration by weight in a range from 0.1% to 80%.

6. The composition according to any of claims 2 to 5, wherein the total concentration by weight of the strain Lactiplantibacillus is in a range from 80 to 20%; and at least one excipient in a concentration by weight in a range from 20% to 80%.

7. The composition according to any of claims 4 to 6, wherein the diluent is in a concentration by weight from 10-30%, the lubricant from 10% to 25% and optionally the thickener in a range from 8-13%.

8. The composition according to any of claims 2 to 7, wherein the composition is administered orally or topically, preferably when the composition is administered orally this is a solid, semi-solid, liquid or gel form.

9. The composition according to any of claim 8, wherein the composition is administered orally and is in the form of capsule, tablet, powder or granule.

10. The composition according to claim 2 to 9, wherein the excipients comprise: silicates, preferably sodium, calcium, potassium or magnesium silicates or hydrophilic fumed silica; magnesium stearate and microcrystalline cellulose.

11. The composition according to any of claims 2 to 10, wherein the composition is a pharmaceutical or cosmetic or nutritional composition.

12. The composition according to any of claims 2 to 11, wherein said composition has a concentration of the strain *Lactiplantibacillus* according to claim 1 of at least 10⁴ of CFU/g, preferably the concentration of the strain *Lactiplantibacillus* is in a range from 10⁶ to 10¹¹.

13. The strain according to claim 1, or the composition according to any of claims 2 to 12, for use in the treatment and/or the prevention of alopecia.

14. The strain according to claim 1, or the composition according to any of claims 2 to 12, for use as a of hair generation promoting agent, of hair growth promoting agent and/or as a hair density promoting agent.

15. A probiotic which comprises the strain according to claim 1 or the composition according to any of claims 2-10, for use in treatment and/or prevention of alopecia or for use as a hair generation promoting agent, of hair growth promoting agent, and/or as a of hair density promoting agent.
